# FASCICULE DE BREVET EUROPEEN

(11) **EP 3 835 404 B1**
(45) Date de publication et mention de la délivrance du brevet: **20.07.2022**
(21) Numéro de dépôt: 20209441.3
(22) Date de dépôt: 24.11.2020
(51) Int. Cl.: C12M 1/107, C12M 1/00, C10L 3/10

(54) **DIGESTEUR COMPRENANT UN FILET DE DÉSULFURATION COMBINÉ À DES CORDES PENDANTES**
FAULBEHÄLTER, DER EIN ENTSCHWEFELUNGSNETZ KOMBINIERT MIT HÄNGESEILEN UMFASST
DIGESTER COMPRISING A COMBINED DESULPHURISATION NET WITH HANGING STRINGS

(30) Priorité: 11.12.2019 FR 1914171
(43) Date de publication de la demande: 16.06.2021
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BERTRANDIAS, Aude, 78350 Les Loges-En-Josas (FR); TRUEBA, Antonio, 78350 Les Loges-En-Josas (FR); SEIWERT, Jacopo, 78350 Les Loges-En-Josas (FR); OLLIER, Jérémy, 38360 Sassenage (FR); VALENTIN, Solène, 38360 Sassenage (FR); FRIMAT, David, 78350 Les Loges-en-Josas (FR)
(74) Mandataire: Air Liquide

(56) Documents cités:
- EP-A1- 1 867 712
- CN-U- 201 825 951
- DE-U1- 20 202 722
- US-A1- 2004 154 982

## Description

La présente invention est relative à une installation et un procédé pour la production de biogaz au moins partiellement désulfuré.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation, par exemple les boues de station d'épuration, fumiers/lisiers, résidus agricoles, déchets alimentaires...

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention et du substrat mais peut également contenir, en moindres proportions de l'eau, de l'azote, de l'hydrogène sulfuré (H₂S), de l'oxygène, ainsi que des composés organiques autres, à l'état de traces, dont le H2S, entre 10 et 50,000 ppmv.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au cœur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bioGNL)...

Selon la composition de la biomasse, le biogaz produit lors de la digestion contient du sulfure d'hydrogène (H₂S) dans des teneurs comprises entre 50 et 50 000 ppm.

Quelle que soit la destination finale de valorisation du biogaz, il s'avère indispensable d'éliminer le sulfure d'hydrogène qui est une impureté toxique et corrosive. De plus, si l'utilisation du biogaz consiste à l'épurer pour injecter du biométhane dans le réseau de gaz naturel, des spécifications strictes limitent la quantité de H₂S autorisée.

Plusieurs méthodes d'élimination du H₂S existent et sont plus ou moins répandues (lits de charbon actif, ajout de composés de fer, absorption physique ou chimique, lavages à l'eau, biofiltres...). L'élimination se fait principalement par adsorption sur lit de charbon actif, à l'extérieur du digesteur. Dans un nombre croissant de digesteurs, l'abattement de l'H2S se fait aussi en partie par injection d'air/air enrichi/O₂ dans le ciel gazeux du digesteur, ce qui constitue une solution in situ. Avec une injection dans le ciel gazeux à faible dose, du soufre solide est formé à partir du H₂S et O₂ (eq. (1)), réalisée par des bactéries sulfo-oxydantes e.g. Thiobacillus. A haute dose d'O₂ injecté, le milieu est acidifié (eq. (2)). La réaction (1) est donc ciblée.

H₂S + 0.5 O₂ → S + H₂O (1)

H₂S + 2O₂ → SO₄²⁻ + 2H + (2)

Les quantités d'injection d'O₂ nécessaires en pratiques sont différentes de celles attendues par la stoechiométrie de l'éq. (1): des doses de 0.3 - 3% d'O₂ par rapport au biogaz généré sont le plus souvent recommandées, avec des doses jusqu'à 12% qui sont parfois évoquées. La demande EP1867712 A divulgue une installation de production de biogaz au moins partiellement désulfurée comprenant un digesteur avec un filet de désulfuration.

Aujourd'hui, l'injection d'air/air enrichi/O₂ in situ n'est pas optimisée et les lits de charbon actif doivent donc être maintenus pour éliminer la totalité du H₂S.

Partant de là, un problème qui se pose est de fournir une installation améliorée favorisant l'élimination plus poussée de l'H₂S.

Une solution de la présente invention est une installation de production de biogaz au moins partiellement désulfuré comprenant un digesteur et/ou un post-digesteur de biomasse, le digesteur et/ou le post-digesteur comprenant:
- Une enceinte 1 dans laquelle a lieu une digestion anaérobie de la biomasse entrainant la production de biogaz 2 et de digestat 3,
- Un moyen d'introduction d'un gaz d'oxydation,
- Un filet de désulfurisation 4 placé à l'horizontal et fixé dans la partie supérieure de l'enceinte, et
- des cordes 5 accrochées au dit filet de désulfurisation et qui pendent jusqu'à l'interface biogaz-digestat .

Nous notons que l'interface biogaz-digestat correspond à l'interface gaz-liquide.

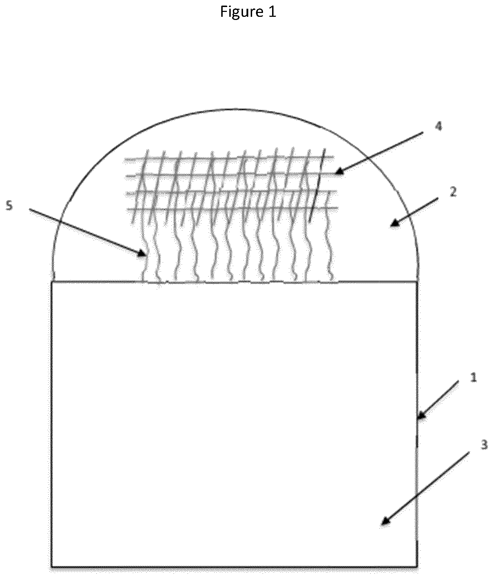

[Fig. 1] La figure 1 est un schéma d'une vue 3D de l'intérieur de l'enceinte du digesteur.

A l'intérieur de l'enceinte du digesteur, lorsque le sulfure d'hydrogène réagit avec l'oxygène, le soufre se fixe notamment sur le filet de désulfurisation et sur les cordes à l'interface gaz-liquide.

Les cordes permettent d'offrir une surface supplémentaire aux bactéries sulfo-oxydantes e.g. Thiobacillus, pour éliminer le soufre à l'endroit où le sulfure d'hydrogène est le plus concentré, c'est-à-dire proche de l'interface liquide-gaz. Une meilleure élimination du soufre est ainsi obtenue.

Selon le cas l'installation selon l'invention peut présenter une ou plusieurs des caractéristiques ci-dessous :
- les cordes sont en polyéthylène.
- les cordes présentent un diamètre compris entre 1 et 50 mm, de préférence entre 2 et 10 mm.
- les cordes présentent une résistance à la rupture comprise entre 50 et 50 000 kg de préférence entre 100 et 500 kg.
- les cordes résistent à des températures comprises entre -30°C et 70°C, de préférence entre 10 et 60°C.
- l'ensemble filet-cordes occupe entre 0,1 et 3% de l'espace gazeux situé entre le digestat et le fond supérieur de l'enceinte.
- des blocs poreux sont accrochés sur le filet et/ou les cordes.
- les blocs poreux comprennent de l'oxyde de fer et/ou du biochar et/ou un charbon actif. De manière générale les blocs poreux peuvent comprendre tout adsorbant connu du sulfure d'hydrogène.
- les cordes sont en polyéthylène.

Le filet pourra être de forme octogonal. Il pourra également être fixé dans l'enceinte en accrochant le filet à des crochets fixés à la paroi interne de l'enceinte.

La présente invention a également pour objet un procédé de production de biogaz au moins partiellement désulfuré mettant en œuvre une installation selon l'invention, comprenant :
- L'injection de biomasse dans le digesteur ;
- L'injection d'un gaz d'oxydation en tête du digesteur ; et
- Le brassage de la biomasse.

Notons que le gaz d'oxydation pourra être de l'oxygène ou de l'air ou de l'air enrichi. Par air enrichi on entend de l'air présentant une teneur en oxygène plus élevée que la teneur en oxygène habituellement comprise dans l'air.

Comme expliqué précédemment, le soufre se fixe sur le filet et les cordes. Au bout d'un certain temps, le soufre solide généré tombe dans le digestat et est évacué avec celui-ci.

La solution selon l'invention permet d'obtenir un flux de biogaz comprenant moins de 200 ppm de sulfure d'hydrogène.

L'invention permet de réduire le coût d'épuration du sulfure d'hydrogène du biogaz de façon efficace, en augmentant la réactivité de l'oxygène déjà injecté avec les produits soufrés grâce à la création d'une surface réactionnelle additionnelle à l'intérieur du ciel gazeux du digesteur, sans besoin complexe en ingénierie.

## Revendications

1. Installation de production de biogaz au moins partiellement désulfuré comprenant un digesteur et/ou un post-digesteur de biomasse, le digesteur et/ou le post-digesteur comprenant:
- Une enceinte (1) dans laquelle a lieu une digestion anaérobie de la biomasse entrainant la production de biogaz (2) et de digestat (3),
- Un moyen d'introduction d'un gaz d'oxydation,
- Un filet de désulfurisation (4) placé à l'horizontal et fixé dans la partie supérieure de l'enceinte, et
- Des cordes (5) accrochées au dit filet de désulfurisation et qui pendent jusqu'à l'interface biogaz-digestat.

2. Installation selon la revendication 1, **caractérisée en ce que** les cordes sont en polyéthylène.

3. Installation selon l'une des revendications 1 ou 2, **caractérisée en ce que** les cordes présentent un diamètre compris entre 1 et 50 mm, de préférence entre 2 et 10 mm.

4. Installation selon l'une des revendications 1 à 3, **caractérisée en ce que** les cordes présentent une résistance à la rupture comprise entre 50 et 50 000 kg de préférence entre 100 et 500 kg.

5. Installation selon l'une des revendications 1 à 4, **caractérisée en ce que** les cordes résistent à des températures comprises entre -30°C et 70°C, de préférence entre 10 et 60°C.

6. Installation selon l'une des revendications 1 à 5, **caractérisée en ce que** l'ensemble filet-cordes occupe entre 0,1 et 3% de l'espace gazeux situé entre le digestat et le fond supérieur de l'enceinte.

7. Installation selon l'une des revendications 1 à 6, **caractérisée en ce que** des blocs poreux sont accrochés sur le filet et/ou les cordes.

8. Installation selon la revendication 7, **caractérisée en ce que** les blocs poreux comprennent de l'oxyde de fer et/ou du biochar et/ou un charbon actif.

9. Procédé de production de biogaz au moins partiellement désulfuré mettant en œuvre une installation selon l'une des revendications 1 à 8, comprenant :
- L'injection de biomasse dans le digesteur ;
- L'injection d'un gaz d'oxydation en tête du digesteur ; et
- Le brassage de la biomasse.

10. Procédé selon la revendication 9, **caractérisé en ce que** le gaz d'oxydation est de l'oxygène ou de l'air ou de l'air enrichi.

## Patentansprüche

1. Anlage zur Produktion von zumindest teilweise entschwefeltem Biogas, umfassend einen Biomasse-Fermenter und/oder einen Biomasse-Nachgärer, wobei der Fermenter und/oder der Nachgärer Folgendes umfassen:
- einen Raum (1), in dem eine anaerobe Fermentation der Biomasse erfolgt, die zur Produktion von Biogas (2) und Gärprodukt (3) führt,
- ein Mittel zum Einleiten eines Oxidationsgases,
- ein Entschwefelungsnetz (4), das horizontal angeordnet und im oberen Teil des Raums befestigt ist, und
- Seile (5), die an dem Entschwefelungsnetz angebracht sind und bis zur Biogas-Gärprodukt-Grenzfläche herabhängen.

2. Anlage nach Anspruch 1, **dadurch gekennzeichnet, dass** die Seile aus Polyethylen bestehen.

3. Anlage nach einem der Ansprüche 1 oder 2, **dadurch gekennzeichnet, dass** die Seile einen Durchmesser zwischen 1 und 50 mm, vorzugsweise zwischen 2 und 10 mm, aufweisen.

4. Anlage nach einem der Ansprüche 1 bis 3, **dadurch gekennzeichnet, dass** die Seile eine Zugfestigkeit zwischen 50 und 50 000 kg, vorzugsweise zwischen 100 und 500 kg, aufweisen.

5. Anlage nach einem der Ansprüche 1 bis 4, **dadurch gekennzeichnet, dass** die Seile gegenüber Temperaturen zwischen -30 °C und 70 °C, vorzugsweise zwischen 10 und 60 °C, widerstandsfähig sind.

6. Anlage nach einem der Ansprüche 1 bis 5, **dadurch gekennzeichnet, dass** die Netz-Seile-Anordnung zwischen 0,1 und 3 % des Gasraums, der sich zwischen dem Gärprodukt und dem oberen Boden des Raums befindet, einnimmt.

7. Anlage nach einem der Ansprüche 1 bis 6, **dadurch gekennzeichnet, dass** an dem Netz und/oder den Seilen poröse Blöcke angebracht sind.

8. Anlage nach Anspruch 7, **dadurch gekennzeichnet, dass** die porösen Blöcke Eisenoxid und/oder Pflanzenkohle und/oder eine Aktivkohle umfassen.

9. Verfahren zur Produktion von zumindest teilweise entschwefeltem Biogas unter Verwendung einer Anlage nach einem der Ansprüche 1 bis 8, umfassend:
- Einspritzen von Biomasse in den Fermenter;
- Einspeisen eines Oxidationsgases am Kopf des Fermenters und
- Umwälzung der Biomasse.

10. Verfahren nach Anspruch 9, **dadurch gekennzeichnet, dass** es sich bei dem Oxidationsgas um Sauerstoff oder Luft oder angereicherte Luft handelt.

## Claims

1. Plant for producing at least partially desulfurized biogas, comprising a biomass digester and/or post-digester, the digester and/or post-digester comprising:
- a chamber (1) in which an anaerobic digestion of the biomass takes place, leading to the production of biogas (2) and of digestate (3),
- a means for introducing an oxidizing gas,
- a desulfurization net (4) placed horizontally and fastened in the upper part of the chamber, and
- ropes (5) attached to said desulfurization net and which hang down to the biogas-digestate interface.

2. Plant according to claim 1, **characterized in that** the ropes are made of polyethylene.

3. Plant according to claim 1 or 2, **characterized in that** the ropes have a diameter of between 1 and 50 mm, preferably between 2 and 10 mm.

4. Plant according to one of claims 1 to 3, **characterized in that** the ropes have a tensile strength of between 50 and 50 000 kg, preferably between 100 and 500 kg.

5. Plant according to one of claims 1 to 4, **characterized in that** the ropes withstand temperatures of between - 30°C and 70°C, preferably between 10°C and 60°C.

6. Plant according to one of claims 1 to 5, **characterized in that** the net-rope assembly occupies between 0.1% and 3% of the gas space located between the digestate and the top end of the chamber.

7. Plant according to one of claims 1 to 6, **characterized in that** porous blocks are attached to the net and/or the ropes.

8. Plant according to claim 7, **characterized in that** the porous blocks comprise iron oxide and/or biochar and/or an activated carbon.

9. Process for producing at least partly desulfurized biogas, using a plant according to one of claims 1 to 8, comprising:
- injecting biomass into the digester;
- injecting an oxidizing gas at the top of the digester; and
- mixing the biomass.

10. Process according to claim 9, **characterized in that** the oxidizing gas is oxygen or air or enriched air.
